# FASCICULE DE BREVET EUROPEEN

(11) **EP 3 602 013 B1**
(45) Date de publication et mention de la délivrance du brevet: **03.08.2022**
(21) Numéro de dépôt: 18709682.1
(22) Date de dépôt: 21.02.2018
(51) Int. Cl.: G01N 21/64, G01N 33/542, G01N 33/58

(54) **METHODE DE DOSAGE DE POLYMERES CATIONIQUES PAR PHOTOLUMINESCENCE RESOLUE EN TEMPS**
VERFAHREN ZUM TESTEN KATIONISCHER POLYMERE DURCH ZEITAUFGELÖSTE FOTOLUMINESZENZ
METHOD FOR ASSAYING CATIONIC POLYMERS BY TIME-RESOLVED PHOTOLUMINESCENCE

(30) Priorité: 27.03.2017 FR 1752540
(43) Date de publication de la demande: 05.02.2020
(73) Titulaire: SPCM SA, 42160 Andrézieux-Bouthéon (FR)
(72) Inventeur: FAVERO, Cédrick, 42160 Andrézieux Bouthéon (FR); SOUZY, Renaud, 42160 Andrézieux Bouthéon (FR); BRAUN, Olivier, 42160 Andrézieux Bouthéon (FR); GUILLARD, Alexis, 42160 Andrézieux Bouthéon (FR)
(74) Mandataire: Cabinet Laurent & Charras
(86) Numéro de dépôt international: PCT/FR2018/050412
(87) Numéro de publication internationale: WO 2018/178528

(56) Documents cités:
- WO-A1-2015/075308
- WO-A1-2016/203119
- James Johnstone ET AL: "SPE-169797-MS Novel Method for Real-Time Monitoring of Scale Control Products at the Site of Use", , 14 mai 2014 (2014-05-14), pages 14-15, XP055257206, Extrait de l'Internet: URL:https://www.onepetro.org/download/conf erence-paper/SPE-169797-MS?id=conference-p aper%2FSPE-169797-MS [extrait le 2016-03-10]
- Thomas Brichart ET AL: "IPTC-17933-MS The Use of Fluorescent Tracers for Inhibitor Concentration Monitoring Useful for Scale Inhibitor Squeeze Evaluation", , 10 décembre 2014 (2014-12-10), XP055257208, Extrait de l'Internet: URL:https://www.onepetro.org/conference-pa per/IPTC-17933-MS [extrait le 2016-03-10]
- Eva F Gudgin Dickson ET AL: "Ultrasensitive bioanalytical assays using time-resolved fluorescence detection", Pharmacology and Therapeutics, 1 janvier 1995 (1995-01-01), pages 207-235, XP055406946, ENGLAND DOI: 10.1016/0163-7258(94)00078-H Extrait de l'Internet: URL:http://ac.els-cdn.com/016372589400078H /1-s2.0-016372589400078H-main.pdf?_tid=fb9 a89fe-99e5-11e7-8be5-00000aab0f6b&acdnat=1 505460050_d10522f32655e62e84d26d77db18472f
- Eva F Gudgin Dickson ET AL: "Ultrasensitive bioanalytical assays using time-resolved fluorescence detection", Pharmacology and Therapeutics, 1 January 1995 (1995-01-01), pages 207-235, XP055407147, ENGLAND DOI: 10.1016/0163-7258(94)00078-H Retrieved from the Internet: URL:http://ac.els-cdn.com/016372589400078H /1-s2.0-016372589400078H-main.pdf?_tid=887 8b44a-9a0e-11e7-b4b5-00000aab0f6c&acdnat=1 505477466_a1240e3ef3043bd5efcb40f52505fd53

## Description

### DOMAINE DE L'INVENTION

La présente invention concerne une méthode de dosage de polymères cationiques. Plus précisément, la présente invention concerne une méthode de dosage de polymères cationiques présents dans un échantillon en utilisant la photoluminescence résolue en temps. Cet échantillon provient d'une eau provenant de procédés de traitement de l'eau ou de boue municipale ou industrielle.

### ETAT DE LA TECHNIQUE

Les polymères cationiques sont utilisés dans des domaines variés tel que le traitement de l'eau industrielle et potable, l'industrie papetière, au cours des différentes étapes de l'extraction minière (forages, rejets miniers, épuration et recyclage), le traitement des boues, ou la récupération assistée du pétrole et du gaz. Dans ces différents domaines il est important pour l'homme du métier de connaitre la concentration des additifs macromoléculaires cationiques (floculant, coagulants, réducteurs de frictions, additifs rhéologiques ...) afin d'éviter tous phénomènes indésirables tels que le rejet dans la nature d'eau contenant des résidus cationiques, les interférences non souhaitées avec d'autres additifs ou tout simplement l'ajustement du dosage en polymère cationique dans les formulations de fluides pétroliers, en application papetière et en traitement des eaux.

De plus, il est fondamental pour l'homme du métier de connaitre la concentration en polymère cationique, car des sous- ou surdosages peuvent conduire à des altérations de leurs performances applicatives dans les formulations telles que la viscosification, la floculation, la compatibilité, la réduction de frictions ainsi que des problèmes environnementaux et des surcouts de production.

De nos jours, différentes techniques analytiques sont utilisées pour déterminer la concentration en polymères cationiques. Comme exemples de techniques, nous pouvons citer la titration colloïdale, la spectroscopie RMN, un test de floculation, ou la titration à l'iode.

Les documents WO 2016/203119 et WO 2015/075308 décrivent des méthodes permettant de doser les inhibiteurs de corrosion ou de dépôt minéraux en utilisant des ions lanthanides.

Toutefois, ces méthodes sont très contraignantes car, pour la plupart, il est indispensable d'avoir un échantillon relativement pur, ce qui nécessite des étapes complexes de purification. De plus, ces méthodes ne permettent pas de déterminer des concentrations très basses en polymères. En effet la plupart de ces méthodes ne peuvent pas détecter des concentrations en dessous de 10 ppm en polymère.

Il existe donc un besoin concernant une méthode de dosage de polymères cationiques dans un échantillon.

### EXPOSE DE L'INVENTION

La présente invention a pour objet une méthode de détermination de la concentration de polymère(s) cationique(s) présent(s) dans un échantillon. Cette méthode comprend les étapes suivantes :
- optionnellement, prétraiter l'échantillon,
- mettre en contact et permettre l'interaction du ou des polymères cationiques présents dans l'échantillon avec une solution révélatrice comprenant des ions lanthanides (III) et au moins un agent de liaisons,
- exciter l'échantillon à une longueur d'onde d'excitation λ_{exc} et détecter, par photoluminescence résolue en temps, un signal provenant des ions lanthanides (III), ayant interagi avec le au moins un agent de liaisons ayant préalablement interagi avec le ou les polymères cationiques, à une longueur d'onde d'émission λₑₘ, et
- déterminer la concentration en polymère(s) cationique(s) de l'échantillon en utilisant le signal détecté à la longueur d'onde d'émission λₑₘ.

L'échantillon mis en œuvre provient d'une eau provenant de procédés de traitement de l'eau ou de boue municipale ou industrielle.

L'agent de liaisons étant choisi parmi les poly(acides carboxyliques) sulfonés, poly(acides carboxyliques) non sulfonés, les dérivés aminés poly(acétiques), les copolymères de monomères sulfonatés, et les macrocyles azotés fonctionnalisés 20 par des groupements acides carboxyliques.

Par « polymère cationique », nous entendons un polymère ou un copolymère ayant une charge globale positive qui contient nécessairement au moins un monomère cationique. En d'autres termes, quand le polymère contient des monomères anioniques et/ou non ioniques, la quantité de charges cationiques est supérieure à la quantité de charges anioniques.

Par « ions lanthanides (III) », nous entendons des ions lanthanides (III) d'un même lanthanide ou de plusieurs lanthanides différents.

La méthode selon la présente invention peut être utilisée pour déterminer la concentration en polymère(s) cationique(s) présent(s) dans des échantillons provenant des procédés de traitement de l'eau ou de boue minérale ou organique.

Cependant, la méthode de la présente invention peut également être utilisée pour déterminer la concentration en polymère(s) cationique(s) présent(s) dans des échantillons provenant de formations souterraines comme les puits de pétrole ou de gaz ; des procédés de traitement de l'eau ou de boue, minérale ou organique ; de la cosmétique ; de la détergence ; de la fabrication du papier ; et ou l'industrie minière.

Il a été découvert de manière totalement surprenante qu'en utilisant la méthode selon l'invention, le signal obtenu par photoluminescence résolue en temps, du produit de l'interaction entre le ou les polymères cationiques et la solution révélatrice comprenant les ions lanthanides, est en corrélation précise avec la concentration en polymère(s) cationique(s) présent(s) dans un échantillon.

Selon l'invention, de manière préférentielle, la mesure de la photoluminescence résolue en temps utilisée est la mesure de la fluorescence résolue en temps.

Cette méthode convient parfaitement pour les polymères ayant un poids moléculaire avantageusement compris entre 1000 et 15 millions g/mol. Sauf indication contraire, par « poids moléculaire » d'un polymère, on entend le poids moléculaire moyen en masse.

Selon l'invention, le polymère peut être un polymère naturel ou un polymère synthétique. Il peut s'agir d'un polymère naturel pouvant être choisi parmi les dérivés des polysaccharides tel que l'amidon, la guar, la cellulose, le dextran ou le xanthane.

Selon l'invention le polymère peut aussi être un polycondensat du type polyamine cationique. Les polyamines cationiques peuvent être obtenues selon le document WO 2009/127893. Le procédé de fabrication utilise principalement 2 familles d'additifs :
- des amines secondaires hydrosolubles du type dimethylamine, diethylamine, dipropylamine, mélange d'aminés secondaires dont les groupements alkyls contiennent de 1 à 3 carbones,
- des époxides difonctionnelles polycondensables en présence d'aminés, telles que l'épibromohydrin, l'épichloroidrin, ou l'épiiodohydrin.

De manière avantageuse, ces polycondensats sont des polyamines à ammonium quaternaire et peuvent être issus de la polycondensation d'épichlorhydrine et de dimethyl amine, de dicyandiamide ou de mélamine et de formol.

Selon l'invention, le polymère cationique peut être obtenu par des procédés bien connus de polymérisation radicalaire conventionnelle en bulk, en solution, en émulsion, en suspension, ou par des techniques de polymérisation radicalaire contrôlée.

De manière avantageuse, l'échantillon comprend au moins un polymère cationique ayant une ou plusieurs charge(s) cationique(s). De manière avantageuse, tous les polymères présents dans l'échantillon comprennent une ou plusieurs charge(s) cationique(s). L'échantillon est avantageusement une composition aqueuse contenant au moins un polymère cationique.

Le polymère cationique peut être un copolymère formé d'au moins deux ou plusieurs monomères. Selon l'invention, le polymère peut avoir une structure linéaire, branchée, réticulée, star (en forme d'étoile) ou comb (en forme de peigne).

Selon l'invention, l'échantillon contient au moins un polymère cationique pouvant être issu de la (co)polymérisation d'au moins un monomère cationique et optionnellement d'au moins un monomère (A) non ionique et/ou d'au moins un monomère anionique ou zwitterionique. Le monomère cationique est avantageusement hydrosoluble.

Le monomère cationique peut être de type acrylamide, acrylique, vinylique, allylique ou maléique et posséder une fonction amine ou ammonium quaternaire. On peut citer, en particulier et de façon non limitative, l'acrylate de diméthylaminoéthyl (ADAME), et le méthacrylate de diméthylaminoéthyle (MADAME) quaternisés ou salifiés, le chlorure de diméthyldiallylammonium (DADMAC), le chlorure d'acrylamido propyltriméthyl ammonium (APTAC) et le chlorure de méthacrylamido propyltriméthyl ammonium (MAPTAC). Les monomères cationiques dérivés de l'acrylamide et portant une chaine hydrophobe décrits dans le document FR 2 868 783 peuvent être utilisés.

Selon un mode de réalisation particulier, le polymère comprend avantageusement entre 1 et 100 mol% de monomère(s) cationique(s), de préférence entre 10 et 100 mol% par rapport au nombre total de moles de monomères du polymère cationique.

Le monomère (A) peut être un monomère non-ionique pouvant notamment être choisi dans le groupe comprenant les monomères vinyliques hydrosolubles, et particulièrement l'acrylamide ; le méthacrylamide, le N-isopropylacrylamide ; le N,N-diméthylacrylamide ; la N-vinylformamide ; l'acryloyl morpholine ; le N,N-diéthyle acrylamide ; le N-tert butyl acrylamide ; le N-tert-octylacrylamide, ; la N-vinylpyrrolidone ; la N-vinyl caprolactame ; la N-vinyl imidazole ; et la diacetone acrylamide.

Le monomère non ionique peut aussi être choisi parmi les monomères de formule :

D-Z-D'

où :
- D est une fonction chimique insaturée polymérisable du type acrylate, méthacrylate, acrylamido, méthacrylamido, vinylique ou allylique,
- D' représente l'hydrogène ou un groupe alkyl (avantageusement en C₁-C₂₂) ou aryl (avantageusement en C₁-C₂₂),
- Z a la structure suivante : -(OE)w-(OP)x-(OBu)z- où :
   - OE, OP, OBu désignent respectivement l'oxyde d'éthylène, l'oxyde de propylène et l'oxyde de butylène,
   - l'arrangement entre les différents motifs d'OE et/ou d'OP et/ou d'OBu peut être statistique, alterné, gradient ou bloc,
   - w, x et z sont des entiers compris entre 0 et 150 et w + x + z ≠ 0.

Selon un mode de réalisation particulier, le polymère comprend avantageusement entre 0 et 99 mol% de monomère(s) non-ionique(s), de préférence entre 0 et 90 mol% par rapport au nombre total de moles de monomères.

Le ou les monomères anioniques peuvent être choisis dans un large groupe. Il s'agit avantageusement d'un monomère hydrosoluble, c'est-à-dire d'un monomère soluble dans l'eau dans les conditions conventionnelles de polymérisation. Ces monomères peuvent présenter des fonctions acryliques, vinyliques, maléiques, fumariques, malonique, itaconique, allyliques. Ils peuvent contenir un groupe carboxylate, phosphonate, phosphate, sulfate, sulfonate, ou un autre groupe à charge anionique. Le monomère anionique peut être sous forme acide ou bien sous forme de sel de métal alcalino-terreux ou de métal alcalin. Des exemples de monomères convenables comprennent l'acide acrylique ; l'acide méthacrylique ; l'acide itaconique ; l'acide crotonique ; l'acide maléique ; l'acide fumarique ; les monomères de type acide fort présentant par exemple une fonction de type acide sulfonique ou acide phosphonique tels que l'acide 2-acrylamido 2-methylpropane sulfonique, l'acide vinylsulfonique, l'acide vinylphosphonique, l'acide allylsulfonique, l'acide allylphosphonique, ou l'acide styrène sulfonique ; et les sels hydrosolubles de ces monomères comme leurs sels de métaux alcalins, de métaux alcalino-terreux, ou d'ammonium.

Selon un mode de réalisation particulier, le polymère comprend avantageusement entre 0 et 49 mol% de monomère(s) anionique(s), de préférence entre 0 et 30 mol%, par rapport au nombre total de moles de monomères.

Selon l'invention, le polymère cationique est très spécifiquement un coagulant et/ou un floculant et/ou un épaississant et/ou un additif réducteur de frictions. Rappelons que les polymères concernés dans la dite invention sont totalement différents des inhibiteurs de dépôts ou de corrosion en terme de fonction, de mécanisme d'action et de morphologie macromoléculaire. En effet, les fonctions et mécanisme d'action des coagulants dans le fluide sont de supprimer les répulsions inter colloïdales et de permettre l'agglomération et la sédimentation des particules colloïdales (minérales et/ou organiques). Les floculants permettent l'agglomération des petites particules et/ou des agrégats coagulés et ainsi la formation de flocs filtrables. Les épaississants permettent d'augmenter la viscosité de fluides aqueux. Quant aux réducteurs de frictions cationiques, leur rôle est de limiter les forces de frictions entre un fluide aqueux et la paroi interne d'une conduite (permettant l'abaissement de la turbulence du fluide). Les fonctions des coagulants, floculants, épaississants et réducteurs de frictions sont donc bien différents des inhibiteurs de dépôts et/ou de corrosion qui s'appuient au contraire sur le renforcement des interactions électrostatiques et/ou électro-stériques entre les particules métalliques/minérales.

Selon l'invention, la solution révélatrice comprend des ions lanthanides (III) et au moins un agent de liaisons.

Les ions lanthanides (III) sont avantageusement choisis parmi les ions europium, terbium, samarium ou dysprosium. Préférentiellement, les ions lanthanide (III) sont des ions europium ou des ions terbium. Dans la solution révélatrice, le lanthanide (III) peut être un sel de lanthanide comme par exemple un halogénure de lanthanide tel que le chlorure d'europium.

L'agent de liaison permettant le lien entre un cation lanthanide et un polymère cationique est avantageusement un agent anionique comportant au moins 2 fonctions anioniques. Préférentiellement, l'agent de liaison comporte plus de 2 fonctions complexantes avec au moins de 4 à 4000 fonctions anioniques. Plus préférentiellement, l'agent de liaison a une structure polymère du type polyélectrolyte ayant une masse molaire en poids avantageusement comprise entre 500 et 100000 g/mol et avec au moins de 10 à 2000 fonctions anioniques par chaînes de polyélectrolyte.

De manière avantageuse, le polyélectrolyte comprend des groupements chimiques possédant des propriétés de fluorescence et permettant une amplification du signal de détection. Pour cela, des monomères fluorescents peuvent être utilisés dans la fabrication du polyélectrolyte. Ainsi, le polyélectrolyte peut être fonctionnalisé par des groupements sulfates, sulfoniques, phosphates, phosphoniques, carboxyliques, imidazoliques, des macrocycles aryliques fonctionnels, des macrocycles hétéroarylique, sous leur forme neutralisée ou acide ainsi que leurs mélanges.

Les agents de liaisons sont avantageusement choisis parmi les poly(acides carboxyliques) sulfonés ou non, les dérivés aminés poly(acétiques), les copolymères de monomères sulfonatés, et les macrocyles azotés fonctionnalisés par des groupements acides carboxyliques. De manière préférentielle, l'agent de liaison est un copolymère d'acide styrène sulfonique et d'acide maléique. Ce polymère est décrit dans le brevet WO2015/092311 sous l'appellation FL1.

Selon un mode particulier de l'invention, la solution révélatrice peut comprendre une ou plusieurs solutions tampons afin d'améliorer le rapport signal sur bruit des échantillons analysés. Parmi les tampons pouvant être utilisés, nous pouvons citer à titre d'exemple, les dérivés d'acide sulfonique, tels que, par exemple HEPES (acide 2- [4- (2-hydroxyéthyl) pipérazin-1 -yl] éthanesulfonique, pKa 7,48), PIPES (1, 4- acide piperazinediethanesulfonic, pKa 6,76), MOPS (3-morpholinopropane-1 - sulfonique, pKa 7,2) et MES (2 - (N-morpholino) éthanesulfonique, pKa 6,15). Préférentiellement, le tampon est l'HEPES. Des solutions révélatrices pouvant être utilisées sont notamment commercialisées par la société Glincs.

La solution révélatrice est avantageusement une solution aqueuse.

Selon un autre mode particulier de l'invention, un ou plusieurs tampons, cités ci-dessus, peuvent être ajoutés à l'échantillon avant la détection du signal à la longueur d'onde d'émission λₑₘ, afin d'améliorer le rapport signal sur bruit et le rapport signal sur bruit de fond des signaux des échantillons détectés.

La quantité d'ions lanthanide (III) introduits dans l'échantillon est avantageusement comprise entre 1 ppm et 10000 ppm, préférentiellement entre 5 ppm et 1000 ppm. La quantité d'ions lanthanide (III) est exprimée en poids par rapport au poids de l'échantillon avant la mise en contact de l'échantillon avec la solution révélatrice.

Selon l'invention, la concentration en polymère(s) cationique(s) est quantifiée, en utilisant une méthode de photoluminescence résolue en temps qui est notamment décrite dans l'article *"*Ultrasensitive bioanalytical assays using time resolved fluorescence detection", Pharmacol. Ther., Vol. 66(2), pages 207-35, 1995. Celle-ci repose sur l'application d'un délai, dit délai d'intégration, entre l'excitation de l'échantillon à analyser et la mesure du signal émis, de manière à s'affranchir des photoluminescences parasites à durée de vie courte. Cette méthode peut être mise en œuvre à température ambiante, notamment à l'aide d'un appareil de type Cary Eclipse de la société Agilent.

La longueur d'onde, qui est utilisée dans l'invention, peut être sélectionnée ou déterminée en étudiant l'excitation maximum dans le spectre d'excitation du produit de l'interaction entre le ou les polymère(s) cationique(s) et la solution révélatrice comprenant des ions lanthanides (III). Par exemple la longueur d'onde d'excitation λ_{exc} peut être comprise entre 200 nm et 600 nm et la longueur d'onde λₑₘ du signal d'émission peut être comprise entre 300 nm et 800 nm.

Le délai d'intégration peut être compris entre 0,001 ms et 10 ms (ms = millisecondes), de préférence entre 0,01 et 5 ms, plus préférentiellement entre 0,1 et 3 ms. Dans certains cas, plus ce délai est long, meilleur est le rapport signal sur bruit, ce qui améliore la fiabilité de la mesure. La durée de récolte des photons peut aller de 5 à 10 ms, par exemple.

L'échantillon peut de manière optionnelle être prétraité avant la détermination de la concentration en polymère(s) cationique(s). Ce prétraitement peut s'avérer utile lorsque l'échantillon comprend des sels, par exemple des sels inorganiques présents dans l'eau de production, ou des particules insolubles. L'eau de production correspond à l'eau récupérée après la séparation eau/hydrocarbures dans un procédé de récupération du pétrole ou du gaz. Ce prétraitement peut aussi s'avérer utile lorsque l'échantillon provient de l'eau qui est issue d'un procédé de fabrication d'une feuille de papier et/ou de carton, d'un procédé de coagulation et/ou floculation d'une eau industrielle et/ou potable, d'un procédé de traitement, coagulation, floculation d'une boue de station de traitement d'eau industrielle et/ou potable.

Selon un mode de réalisation de l'invention, la méthode comprend une étape de purification de l'échantillon avant l'addition de la solution révélatrice comprenant les ions lanthanides (III) et les agents de liaisons. Ainsi, l'échantillon peut être purifié afin d'éliminer des substances et/ou des composés pouvant interférer avec le signal mesuré à la longueur d'onde d'émission λₑₘ. Par exemple, un pré-nettoyage peut aider à minimiser le bruit de fond provoqué par les composants de l'échantillon. Parmi les procédés de purification pouvant être utilisés dans le cadre de l'invention, nous pouvons citer à titre d'exemple, la centrifugation, la chromatographie d'exclusion de taille, le nettoyage avec des cartouches d'extraction en phase solide (SPE), les techniques de dialyse, les méthodes d'extraction pour l'élimination des hydrocarbures, la filtration, la microfiltration, l'ultrafiltration, la nanofiltration, la centrifugation sur membrane et/ou d'autres méthodes permettant de séparer les espèces polymériques ayant un petit poids moléculaire (avantageusement inférieur à 1000 g/mol).

Selon un mode de réalisation de l'invention, la concentration en sel de l'échantillon peut être modifiée et/ou les particules insolubles peuvent être éliminées avant l'addition de la solution révélatrice comprenant les ions lanthanides (III). La modification de la concentration en sel de l'échantillon peut se traduire par une diminution ou une augmentation de la concentration en sel avant l'addition de la solution révélatrice comprenant les ions lanthanides (III).

Selon un mode particulier de l'invention, si l'échantillon est trop visqueux à cause d'une concentration initiale en polymère(s) cationique(s) trop importante, l'échantillon peut être dilué avant l'addition de la solution révélatrice comprenant les ions lanthanides (III). Les diluants peuvent être choisis parmi l'eau, des solutions tampons aqueuses, des solutions salines saturées ou non en sels, ou leurs mélanges.

Selon un mode particulier de l'invention, une ou plusieurs des étapes de prétraitement ci-dessus peuvent être effectuées sur un échantillon avant la mesure de la concentration en polymère(s) cationique(s) d'un échantillon. Par exemple, avant la mesure, l'échantillon peut être purifié et/ou dilué.

Selon un mode particulier de l'invention, la valeur du pH de l'échantillon est ajustée à un niveau approprié. Le pH de l'échantillon est avantageusement compris entre 3 et 10, de préférence entre 5 et 8. Tout tampon approprié qui ne perturbe pas de manière significative la détection du signal échantillon, peut être utilisé. Des exemples de tampons sont donnés ci-dessus, mais d'autres tampons peuvent également être utilisés.

Pour déterminer la concentration en polymères cationiques, une courbe standard ou des points standards peuvent être préparés avant d'utiliser la méthode de détermination selon l'invention. La concentration en polymères cationiques peut être calculée à partir du signal en se référant à la courbe standard ou à des points standards prédéterminés. D'une manière alternative l'instrument de mesure peut être pré-calibré.

Le protocole ci-dessous, de dosage des polymères cationiques, peut être suivi :
1) On prépare des solutions de concentrations connues en polymère cationique par dilution successive d'une solution mère de polymère cationique avec de l'eau ayant avantageusement les caractéristiques (notamment la salinité et la conductivité) de l'échantillon X de concentration inconnue. Les échantillons de chacune des séries, sont ensuite dilués avec une solution révélatrice de lanthanide et analysés par Fluorescence en Temps Résolu (FTR). Les paramètres de mesure ainsi que les longueurs d'onde d'émission, d'excitation sont réglées en fonction de la nature du lanthanide.
2) Pour chacune des séries, les Intensités Signal FTR vs concentration molaire sont extrapolées et une courbe de calibration Intensité FTR vs concentration est élaborée.
3) L'échantillon X est ensuite dilué avec la solution révélatrice. On mesure l'intensité FTR.
4) Après corrélation de l'intensité FTR de l'échantillon X avec la droite de calibration de concentration cationique en 2), la concentration en polymère cationique préalablement inconnue de l'échantillon X est déduite.

Les étapes de dilution peuvent être réalisées par addition d'eau.

Cependant, lorsque l'échantillon provient d'une eau de production, toutes les étapes de dilution peuvent être réalisées avec une saumure ayant les mêmes caractéristiques de conductivité et de salinité que l'eau de production, même pour les polymères standards.

Il est important de noter qu'il est aussi possible de déterminer la densité de charge cationique du polymère par extrapolation de la pente de la courbe Intensité Signal FTR vs Taux de Dilution et corrélation avec la courbe de variation de la pente d'intensité vs dilution de solution mères de polymère de cationicité connue. De manière générale, toute technique de photoluminescence en temps résolu peut substituer la FTR, notamment pour les étapes 1) à 4) ci-dessus.

L'invention et les avantages qui en découlent ressortiront mieux des figures et des exemples suivants donnés afin d'illustrer l'invention et non de manière limitative.

### DESCRIPTION DES FIGURES

La figure 1 illustre le graphe de l'intensité du signal à la longueur d'onde d'émission λₑₘ en fonction de la concentration en poly(DADMAC).
La figure 2 illustre le graphe de l'intensité du signal à la longueur d'onde d'émission λₑₘ en fonction de la concentration en polyamine cationique.
La figure 3 illustre le graphe de l'intensité du signal à la longueur d'onde d'émission λₑₘ en fonction de la concentration en poly(AM-co-ADAME quat).

### EXEMPLES DE REALISATION DE L'INVENTION

Les abréviations suivantes sont utilisées :
DADMAC : Diallyl dimethyl amonium chlorure, [(CH₃)₂N⁺(CH₂CH=CH₂)_{2]}Cl⁻
AM : Acrylamide, CH₂=CH-C(=O)-NH₂
ADAME quat : Acrylate de triméthylamonium éthyl chlorure, CH₂=CH-C(=O)-O-(CH₂CH₂)-N⁺(CH₃)₃.Cl⁻
λₑₘ : Longueur d'onde d'émission
λ_{exc} : Longueur d'onde d'excitation

### Exemple 1 - Dosage de la concentration résiduelle d'un polymère cationique coagulant dans une eau industrielle de rejet

Cet exemple porte sur le dosage de la concentration résiduelle d'un coagulant cationique du type poly(DADMAC) présent dans une eau industrielle de rejet (conductivité de 385 µS.cm⁻¹ à 20°C).

### a) Préparation des solutions témoins

On prépare des solutions de concentrations connues de poly(DADMAC) (nom commercial : Floquat FL4440 - SNF) par dilution successive d'une solution mère de polymère avec de l'eau ayant la même composition saline chimique que l'eau industrielle (Tableau 1).

**Tableau 1 : concentration des solutions témoins de poly(DADMAC)**

| **Référence** | **Composition Chimique** | **Concentration (ppm, en poids par rapport au poids de la solution)** |
|---|---|---|
| A0 | - | 0 |
| A1 | poly(DADMAC) | 0,4 |
| A2 | poly(DADMAC) | 0,8 |
| A3 | poly(DADMAC) | 1 |
| A4 | poly(DADMAC) | 1,5 |
| A5 | poly(DADMAC) | 2 |
| A6 | poly(DADMAC) | 4 |
| A7 | poly(DADMAC) | 6 |
| A8 | poly(DADMAC) | 8 |
| A9 | poly(DADMAC) | 10 |
| A10 | poly(DADMAC) | 15 |
| A11 | poly(DADMAC) | 20 |

Les caractéristiques de l'eau de dilution sont :
Concentratoin en fer : 103 µg/L
Concentration en chlorite : 0,13 mg.L⁻¹
Concentration en sodium : 145 mg.L⁻¹
Concentration en aluminium : 133 µg.L⁻¹
Turbidité : 0,3 NFU

### b) Complexation avec des solutions révélatrices

Chacune des solutions listées dans le tableau 1 sont diluées au 9/10 dans une solution révélatrice d'europium associé à un copolymère d'acide styrène sulfonique et d'acide maléique commercialisée par la société Glincs.

### c) Mesure de Luminescence en Temps Résolu

Les mesures sont effectuées à 20°C dans une cuvette en quartz sur un spectromètre du type Cary Eclipse Fluorescence Spectrophotometer de la société Agilent ayant pour caractéristiques :
- Lampe flash au Xenon de 75 kW
- Monochromateurs Czerny-Turner
- Détecteur PM à 800 V
- Δₚᵤₗₛₑ = 2 µS

Les spectres d'excitation des échantillons sont réalisés entre 200 et 450 nm. La longueur d'onde d'émission est λₑₘ = 545 nm.

Les paramètres de mesure sont réglés de la façon suivante :
- Délai : 0,1 ms
- Durée de récolte photonique : 5 ms
- Fréquence de la lampe : 100 Hz
- Nombre de flash : 1

L'analyse est lancée à l'aide du logiciel contrôlant le spectrofluorimètre.

### d) Courbe de calibration

Les spectres d'excitation des différentes solutions du Tableau 1 sont réalisés à λ_{exc} = 260 nm et λₑₘ = 610 nm.

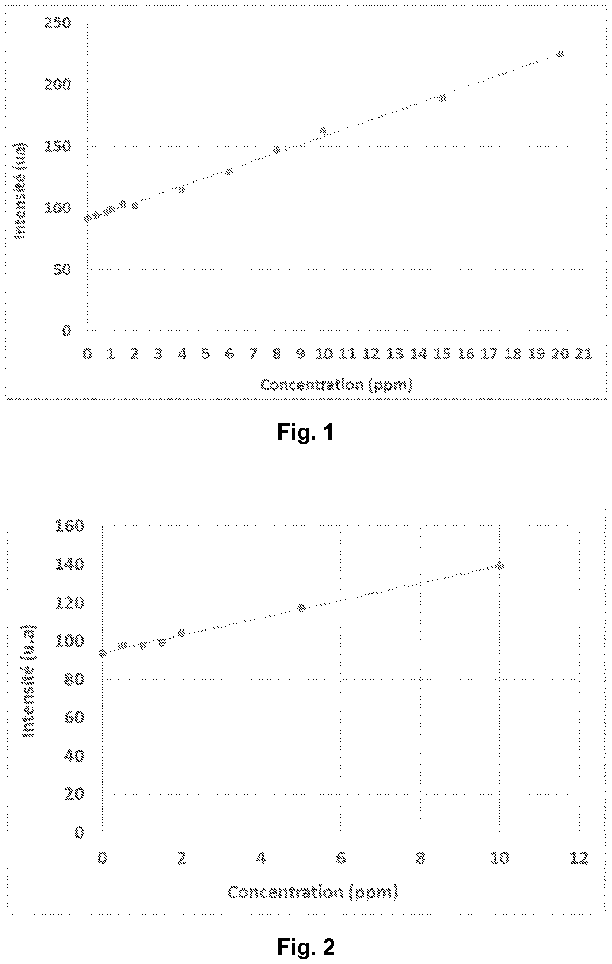

Les intensités des pics en fonction de la concentration sont présentées dans la Figure 1.

### e) Dosage de la concentration en coagulant d'un échantillon prélevé dans l'eau industrielle de rejet

Un échantillon de concentration inconnue a été prélevé dans un volume d'eau industrielle rejetée.

Cette solution est diluée au 9/10 dans une solution révélatrice d'europium associé à un copolymère d'acide styrène sulfonique et d'acide maléique commercialisée par la société Glincs.

L'intensité mesurée est de 93,6 u.a.. Après extrapolation avec la droite de calibration de la Figure 1, on déduit que la concentration en coagulant est de 0,35 ppm en poids, par rapport au poids de l'échantillon.

Cet exemple démontre qu'il est possible de doser la concentration en polymère cationique par la technique de fluorescence en temps résolu.

### Exemple 2 - Dosage résiduel d'une polyamine cationique dans une eau industrielle de lavage de granulats

Cet exemple porte sur le dosage de la concentration résiduelle d'un coagulant cationique du type poly(amine quaternaire) présent dans une eau industrielle issue du lavage de granulat.

### a) Préparation des solutions témoins

On prépare les solutions suivantes de différentes concentrations par dilution successive d'une solution mère de poly(amine cationique) (nom commercial : Floquat FL2250 - SNF) avec de l'eau ayant les même caractéristiques salines et de conductivité que l'eau de production.

**Tableau 2 : Composition des solutions témoins de poly(amine cationique)**

| **Référence** | **Composition Chimique** | **Concentration (ppm, en poids par rapport au poids de la solution)** |
|---|---|---|
| B0 | - | 0 |
| B1 | poly(amine cationique) | 0,5 |
| B2 | poly(amine cationique) | 1 |
| B3 | poly(amine cationique) | 1,5 |
| B4 | poly(amine cationique) | 2 |
| B5 | poly(amine cationique) | 5 |
| B6 | poly(amine cationique) | 10 |

Les caractéristiques de l'eau de dilution sont :
Conductivité : 504 µS.cm⁻¹
Concentration en fer : 185 µg/L

### b) Complexation avec des solutions révélatrices

Chacune des solutions listées dans le tableau 2 sont diluées au 9/10 dans une solution révélatrice d'europium associé à un copolymère d'acide styrène sulfonique et d'acide maléique commercialisée par la société Glincs.

### c) Mesure de Luminescence en Temps Résolu

Les mesures sont effectuées à 20°C dans une cuvette en quartz sur un spectromètre du type Cary Eclipse Fluorescence Spectrophotometer de la société Agilent ayant pour caractéristiques :
- Lampe flash au Xenon de 75 kW
- Monochromateurs Czerny-Turner
- Détecteur PM à 800 V
- Δₚᵤₗₛₑ = 2 µS

Les spectres d'excitation des différentes solutions du Tableau 2 sont réalisés à λ_{exc} = 260 nm et λₑₘ = 610 nm.

Les paramètres de mesure sont réglés de la façon suivante :
- Délai : 0,1 ms
- Durée de récolte photonique : 5 ms
- Fréquence de la lampe : 100 Hz
- Nombre de flash : 1

L'analyse est lancée à l'aide du logiciel contrôlant le spectrofluorimètre.

### d) Courbe de calibration

Les spectres d'excitation des différentes solutions du Tableau 2 sont réalisés à λ_{exc} = 260 nm et λₑₘ = 610 nm.

La variation linéaire de l'intensité des pics en fonction de la concentration sont présentées dans la Figure 2.

### e) Dosage de la concentration d'un échantillon de poly(amine cationique)

Un échantillon de concentration inconnue a été prélevé dans un volume d'eau industrielle de lavage de granulat. Les macro-particules en suspensions sont préalablement filtrées.

Cette solution est diluée au 9/10 dans une solution révélatrice d'europium associé à un copolymère d'acide styrène sulfonique et d'acide maléique commercialisée par la société Glincs.

L'intensité mesurée est de 94,2 u.a.. Après extrapolation avec la droite de calibration de la Figure 1, on déduit que la concentration en coagulant est de 0,21 ppm.

Cet exemple démontre qu'il est possible de doser la concentration des polymères cationiques ayant de nature chimique différente et autre que les poly(DADMAC) par la technique de fluorescence en temps résolu.

### Exemple 3 - Dosage résiduel d'un copolymère floculant dans une eau issue de la floculation de boues industrielles

Cet exemple porte sur le dosage de la concentration résiduelle d'un floculant cationique du type poly(AM-co-ADAME quat) de très haute masse molaire en poids présent dans une eau industrielle issue de la floculation de boue industrielle d'une station d'épuration.

### a) Préparation des solutions témoins

On prépare les solutions suivantes de différentes concentrations par dilution successive d'une solution mère de poly(AM-co-ADAME quat) (nom commercial : Flopaam FO 4650 SSH - SNF) avec de l'eau ayant les même caractéristiques salines et de conductivité que l'eau de production.

**Tableau 3 : Composition des solutions témoins de poly(AM-co-ADAME quat)**

| **Référence** | **Composition Chimique** | **Concentration (ppm, en poids par rapport au poids de la solution)** |
|---|---|---|
| C0 | - | 0 |
| C1 | poly(AM-co- ADAME quat) | 0,5 |
| C2 | poly(AM-co- ADAME quat) | 1 |
| C3 | poly(AM-co- ADAME quat) | 2 |
| C4 | poly(AM-co- ADAME quat) | 3 |
| C5 | poly(AM-co- ADAME quat) | 5 |
| C6 | poly(AM-co- ADAME quat) | 7 |
| C7 | poly(AM-co-ADAME quat) | 10 |
| C8 | poly(AM-co-ADAME quat) | 12 |
| C9 | poly(AM-co-ADAME quat) | 14 |
| C10 | poly(AM-co-ADAME quat) | 18 |
| C11 | poly(AM-co-ADAME quat) | 20 |
| C11 | poly(AM-co-ADAME quat) | 40 |
| C12 | poly(AM-co-ADAME quat) | 60 |
| C13 | poly(AM-co-ADAME quat) | 80 |
| C14 | poly(AM-co-ADAME quat) | 100 |
| C14 | poly(AM-co-ADAME quat) | 200 |

Les caractéristiques de l'eau de dilution sont :
Conductivité : 750 µS.cm⁻¹
Concentratin en fer : 215 µg/L

### b) Complexation avec des solutions révélatrices

Chacune des solutions listées dans le Tableau 3 sont diluées au 9/10 dans une solution révélatrice d'europium associé à un copolymère d'acide styrène sulfonique et d'acide maléique.

### c) Mesure de Luminescence en Temps Résolu

Les mesures sont effectuées à 20°C dans une cuvette en quartz sur un spectromètre du type Cary Eclipse Fluorescence Spectrophotometer de la société Agilent ayant pour caractéristiques :
- Lampe flash au Xenon de 75 kW
- Monochromateurs Czerny-Turner
- Détecteur PM à 800 V
- Δₚᵤₗₛₑ = 2 µS

Les spectres d'excitation des différentes solutions du Tableau 3 sont réalisés à λ_{exc} = 260 nm et λₑₘ = 610 nm.

Les paramètres de mesure sont réglés de la façon suivante :
- Délai : 0,1 ms
- Durée de récolte photonique : 5 ms
- Fréquence de la lampe : 100 Hz
- Nombre de flash : 1

L'analyse est lancée à l'aide du logiciel contrôlant le spectrofluorimètre.

### d) Courbe de calibration

Les spectres d'excitation des différentes solutions du Tableau 3 sont réalisés à λ_{exc} = 260 nm et λₑₘ = 610 nm.

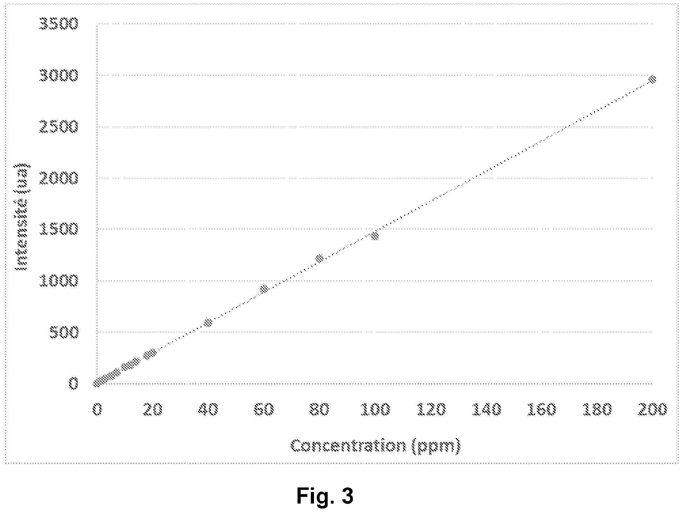

La variation linéaire de l'intensité des pics en fonction de la concentration sont présentées dans la Figure 3.

### e) Dosage de la concentration d'un échantillon de poly(AM-co-ADAME quat)

Un échantillon de concentration inconnue a été prélevé dans un volume d'eau industrielle issue de la floculation de boue industrielle de la station d'épuration. Les macro-particules en suspensions sont préalablement filtrées.

Cette solution est diluée au 9/10 dans une solution révélatrice d'europium associé à un copolymère d'acide styrène sulfonique et d'acide maléique commercialisée par la société Glincs.

L'intensité mesurée est de 83,31 u.a.. Après extrapolation avec la droite de calibration de la Figure 3, on déduit que la concentration en floculant est de 5,33 ppm.

Cet exemple démontre qu'il est possible de doser la concentration des polymères cationiques de très hautes masses molaires moyennes en poids du type floculant par la technique de fluorescence en temps résolu.

## Revendications

1. Méthode de détermination de la concentration en polymères cationiques présent dans un échantillon, selon les étapes suivantes :
- mettre en contact et permettre l'interaction du ou des polymères cationiques présents dans l'échantillon avec une solution révélatrice comprenant des ions lanthanides (III) et au moins un agent de liaisons,
- exciter l'échantillon à une longueur d'onde d'excitation λ_{exc} et détecter, par photoluminescence résolue en temps, un signal provenant des ions lanthanides (III) ayant interagi avec le au moins un agent de liaisons ayant préalablement interagi avec le ou les polymères cationiques, à une longueur d'onde d'émission λₑₘ, et
- déterminer la concentration en polymère cationique de l'échantillon en utilisant le signal détecté à la longueur d'onde d'émission λₑₘ,
l'échantillon provenant d'une eau provenant de procédés de traitement de l'eau ou de boue municipale ou industrielle,
l'agent de liaisons étant choisi parmi les poly(acides carboxyliques) sulfonés, poly(acides carboxyliques) non sulfonés, les dérivés aminés poly(acétiques), les copolymères de monomères sulfonatés, et les macrocyles azotés fonctionnalisés par des groupements acides carboxyliques.

2. Méthode selon l'une des revendications précédentes, ***caractérisée* en ce que** la photoluminescence résolue en temps est la fluorescence résolue en temps.

3. Méthode selon l'une des revendications précédentes, ***caractérisée* en ce que** l'échantillon contient au moins un polymère issu de la polymérisation d'au moins un monomère cationique et optionnellement, d'au moins un monomère (A) non ionique et/ou d'au moins un monomère anionique ou zwitterionique.

4. Méthode selon l'une des revendications précédentes, ***caractérisée* en ce que** les ions lanthanides sont choisis parmi les ions europium, terbium, samarium et dysprosium.

5. Méthode selon l'une des revendications précédentes, ***caractérisée* en ce que** les ions lanthanides sont des ions europium ou des ions terbium.

6. Méthode selon l'une des revendications précédentes, ***caractérisée* en ce qu'**une quantité d'ions lanthanide (III) comprise entre 1 ppm et 10000 ppm est introduite dans l'échantillon, en poids par rapport au poids de l'échantillon avant la mise en contact de l'échantillon avec la solution révélatrice.

7. Méthode selon l'une des revendications précédentes, ***caractérisée* en ce que** la longueur d'onde d'excitation λ_{exc} est comprise entre 200 nm et 600 nm,
et **en ce que** la longueur d'onde λₑₘ du signal d'émission est comprise entre 300 nm et 800 nm.

8. Méthode selon l'une des revendications précédentes, ***caractérisée* en ce que** l'agent de liaisons est un agent anionique comportant au moins 2 fonctions anioniques.

9. Méthode selon l'une des revendications précédentes, ***caractérisée* en ce que** la méthode comprend une étape de purification de l'échantillon avant l'addition de la solution révélatrice comprenant les ions lanthanides (III).

## Patentansprüche

1. Verfahren zur Bestimmung der Konzentration kationischer Polymere in einer Probe, mit den folgenden Schritten:
- das oder die in der Polymer vorhandene kationischen Polymere mit einer Entwicklerlösung in Kontakt bringen, die Lanthan (III)- Ionen und mindestens einen Haftvermittler enthält,
- Anregen der Probe auf eine Anregungswellenlänge λ*_{exc}* und Erkennen eines Signals durch zeitaufgelöste Photolumineszenz, das aus den Lanthan (III)- Ionen stammt, die mit dem mindestens einen Haftvermittler interagierten, der vorher mit dem oder den kationischen Polymeren interagierte, mit einer Emissionswellenlänge λ*ₑₘ,* und
- Bestimmung der Konzentration an kationischen Polymeren der Probe unter Verwendung des bei der Emissionswellenlänge λ*ₑₘ* bestimmten Signals,
die Probe stammt dabei aus dem Wasser aus Wasseraufbereitungsverfahren oder aus Gemeinde- oder industriellen Kläranlagen,
der Haftvermittler wird ausgewählt aus sulfonierten Poly(Carboxylsäuren), nichtsulfonierten Poly(Carboxylsäuren), Amino-Poly(essigsäure)- Derivaten, Copolymeren von Sulfonatmonomeren und stickstoffhaltige Makrozylen, funktionalisiert durch Carboxylsäure - Gruppen.

2. Verfahren nach einem der vorangehenden Ansprüche, ***dadurch gekennzeichnet, dass*** die zeitaufgelöste Photolumineszenz, die zeitaufgelöste Lumineszenz ist.

3. Verfahren nach einem der vorangehenden Ansprüche, ***dadurch gekennzeichnet, dass*** die Probe mindestens ein Polymer aus der Polymerisierung mindestens eines kationischen Monomers enthält und optional mindestens ein nicht-ionisches Monomer (A) und/ oder mindestens ein anionisches oder zwitterionisches Monomer enthält.

4. Verfahren nach einem der vorangehenden Ansprüche, ***dadurch gekennzeichnet, dass*** die Lanthanionen ausgewählt werden aus Europium-, Terbium-, Samarium-und Dysprosium - Ionen.

5. Verfahren nach einem der vorangehenden Ansprüche, ***dadurch gekennzeichnet, dass*** die Lanthanionen Europium- oder Terbiumionen sind.

6. Verfahren nach einem der vorangehenden Ansprüche, ***dadurch gekennzeichnet, dass*** eine Menge an Lanthan (III) - Ionen zwischen 1 ppm und 10000 ppm in die Probe eingeführt werden, in Gewicht bezogen auf das Gewicht der Probe bevor die Probe mit der Entwicklerlösung in Kontakt gebracht wurde.

7. Verfahren nach einem der vorangehenden Ansprüche, ***dadurch gekennzeichnet, dass*** die Anregungswellenlänge λ*_{exc}* zwischen 200 nm und 600 nm liegt,
und dass die Wellenlänge λ*ₑₘ* des Emissionssignals zwischen 300 nm und 800 nm liegt.

8. Verfahren nach einem der vorangehenden Ansprüche, ***dadurch gekennzeichnet, dass*** der Haftvermittler ein anionisches Mittel ist, das mindestens 2 anionische Funktionen enthält.

9. Verfahren nach einem der vorangehenden Ansprüche, ***dadurch gekennzeichnet, dass*** das Verfahren einen Schritt der Reinigung der Probe vor der Zugabe der Entwicklerlösung mit den Lanthan (III) - Ionen enthält.

## Claims

1. Method for determining the concentration of cationic polymers present in a sample, according to the following steps:
- bringing the cationic polymer(s) present in the sample into contact with, and enabling the interaction thereof with, a developer solution comprising lanthanide (III) ions and at least one bonding agent,
- exciting the sample at an excitation wavelength λ_{exc} and detecting, by time-resolved photoluminescence, a signal from the lanthanide (III) ions that have interacted with at least one bonding agent having previously interacted with the cationic polymer(s) at an emission wavelength λₑₘ, and
- determining the cationic polymer concentration of the sample using the signal detected at the emission wavelength λₑₘ,
the sample originating from water obtained from water treatment processes or from municipal or industrial sludge,
the bonding agent is selected from sulfonated poly(carboxylic acids), non-sulfonated poly(carboxylic acids), poly(acetic)amine derivatives, copolymers of sulfonated monomers, and nitrogenous macrocycles functionalized with carboxylic acid groups.

2. Method according to one of the previous claims ***characterized in that*** the time-resolved photoluminescence is time-resolved fluorescence.

3. Method according to one of the previous claims, ***characterized in that*** the sample contains at least one polymer resulting from the polymerization of at least one cationic monomer and, optionally, at least one non-ionic monomer (A) and/or at least one anionic or zwitterionic monomer.

4. Method according to one of the previous claims ***characterized in that*** the lanthanide ions are chosen from europium, terbium, samarium or dysprosium ions.

5. Method according to one of the previous claims ***characterized in that*** the lanthanide ions are europium or terbium ions.

6. Method according to one of the previous claims ***characterized in that*** an amount of lanthanide (III) ions between 1 ppm and 10,000 ppm is added to the sample by weight based on the weight of the sample before the sample comes into contact with the developer solution.

7. Method according to one of the previous claims, ***characterized in that*** the excitation wavelength λ_{exc} is between 200 nm and 600 nm,
and the emission signal wavelength λₑₘ is between 300 nm and 800 nm.

8. Method according to one of the previous claims, ***characterized in that*** the bonding agent is an anionic agent comprising at least 2 anionic functions.

9. Method according to one of the previous claims, ***characterized in that*** the method comprises a sample purification step prior to adding the developer solution comprising the lanthanide (III) ions.
